# EUROPÄISCHE PATENTSCHRIFT

(11) **EP 1 228 041 B1**
(45) Veröffentlichungstag und Bekanntmachung des Hinweises auf die Patenterteilung: **15.10.2003**
(21) Anmeldenummer: 00954447.9
(22) Anmeldetag: 07.07.2000
(51) Int. Cl.: C07D 209/12, A61K 31/404, A61P 43/00, C07D 403/06, C07D 409/06

(54) **INDOLDERIVATE UND DEREN VERWENDUNG ALS 5HT2A LIGANDEN**
INDOLE DERIVATIVES AND THEIR USE AS 5HT2A LIGANDS
DERIVES INDOLIQUES ET LEUR UTILISATION COMME LIGANDS 5HT2A

(30) Priorität: 22.07.1999 DE 19934432
(43) Veröffentlichungstag der Anmeldung: 07.08.2002
(73) Patentinhaber: MERCK PATENT GmbH, 64293 Darmstadt (DE)
(72) Erfinder: BÖTTCHER, Henning, D-64287 Darmstadt (DE); MÄRZ, Joachim, D-64521 Gro -Gerau (DE); GREINER, Hartmut, D-64331 Weiterstadt (DE); HARTING, Jürgen, D-64287 Darmstadt (DE); BARTOSZYK, Gerd, D-64331 Weiterstadt (DE); SEYFRIED, Christoph, D-64342 Seeheim-Jugenheim (DE); VAN AMSTERDAM, Christoph, D-64295 Darmstadt (DE)
(86) Internationale Anmeldenummer: EP0006463
(87) Internationale Veröffentlichungsnummer: WO01007434

(56) Entgegenhaltungen:
- EP-A- 0 599 240
- WO-A-99/11641

## Beschreibung

Die Erfindung betrifft Verbindungen der Formel I 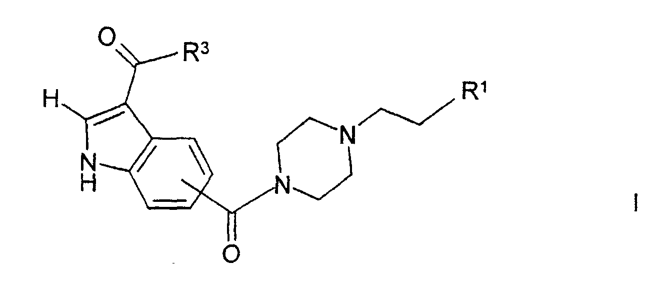 worin
- R¹: einen unsubstituierten oder durch R² und/oder R⁴ substituierten Phenylrest
oder Het¹,
- R², R⁴, R⁵, R⁶: jeweils unabhängig voneinander Hal, A, OA, OH, CN oder Acyl,
- R³: einen unsubstituierten oder durch R⁵ und/oder R⁶ substituierten Phenylrest
oder Het²,
- Het¹: ein- oder zweikerniges unsubstituiertes oder ein- oder zweifach durch Hal, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
- Het²: unsubstituiertes oder ein- oder zweifach durch Hal, A, OA, ungesättigtes heterocyclisches Ringsystem, welches eines oder zwei gleiche oder verschiedene Heteroatome wie Stickstoff, Sauerstoff und Schwefel enthält,
- A: Alkyl mit 1-6 C-Atomen,
- Hal: F, Cl, Br oder I,
bedeuten,
sowie deren physiologisch unbedenklichen Salze und Solvate.

Der Erfindung lag die Aufgabe zugrunde, neue Verbindungen mit wertvollen Eigenschaften aufzufinden, insbesondere solche, die zur Herstellung von Arzneimitteln verwendet werden können

Es wurde gefunden, dass die Verbindungen der Formel I und ihre physiologisch unbedenklichen Salze und Solvate bei guter Verträglichkeit wertvolle pharmakologische Eigenschaften besitzen, da sie Wirkungen auf das Zentralnervensystem besitzen. Die Verbindungen weisen eine starke Affinität zu 5-HT_{2A}-Rezeptoren auf, weiterhin zeigen sie 5-HT_{2A}-Rezeptor-antagonistische Eigenschaften.

Zum in-vitro Nachweis der Affinität zu 5-HT_{2A}-Rezeptoren kann beispielsweise folgender Test (Beispiel A1) herangezogen werden. Die 5-HT_{2A} Rezeptoren werden sowohl [³H]Ketanserin (eine Substanz, bekannt für ihre Affinität zum Rezeptor) als auch der Testverbindung ausgesetzt. Die Abnahme der Affinität von [³H]Ketanserin zum Rezeptor ist ein Anzeichen für die Affinität der Testsubstanz zum 5-HT_{2A} Rezeptor. Der Nachweis erfolgt analog der Beschreibung von J.E. Leysen et al., Molecular Pharmacology, 1982, 21: 301-314 oder wie z.B. auch in EP 0320983 beschrieben.

Die Wirksamkeit der erfindungsgemäßen Verbindungen als 5-HT_{2A} Rezeptor-Antagonisten kann in vitro analog W. Feniuk et al., Mechanisms of 5-hydroxytryptamine-induced vasoconstriction, in: The Peripheral Actions of 5-Hydroxytryptamine, ed. Fozard JR, Oxford University Press, New York, 1989, p.110, gemessen werden. So wird die Kontraktilität der Rattenschwanzarterie, hervorgerufen durch 5-Hydroxytryptamin, durch 5-HT_{2A} Rezeptoren vermittelt. Für das Testsystem werden Gefäßringe, präpariert aus der ventralen Rattenschwanzarterie, in einem Organbad mit einer sauerstoffgesättigten Lösung einer Perfusion unterzogen. Durch Eintrag ansteigender Konzentrationen an 5-Hydroxytryptamin in die Lösung erhält man eine Antwort auf die kumulative Konzentration an 5-HT. Danach wird die Testverbindung in geeigneten Konzentrationen in das Organbad gegeben und eine zweite Konzentrationskureve für 5-HT gemessen. Die Stärke der Testverbindung auf die Verschiebung der 5-HT induzierten Konzentrationskurve zu höheren 5-HT Konzentrationen ist ein Maß für die 5-HT_{2A}-Rezeptor-anatgonistische Eigenschaft in vitro.

Die 5-HT_{2A}-antagonistische Eigenschaft kann in vivo analog M.D.Serdar et al., Psychopharmacology, 1996, 128: 198-205, bestimmt werden.

Andere Verbindungen, die ebenfalls 5-HT₂-antagonistische Wirkungen zeigen, sind beispielweise in der EP 0320983 beschrieben.
Ähnliche Piperazinderivate mit antiarrhythmischen Eigenschaften sind z.B. in der EP 0431945 offenbart. Andere Indolcarbonylderivate mit analgetischen Eigenschaften sind in der EP 0599240 beschrieben. In der EP 0624584 sind Piperazinderivate als Calmodolin-Inhibitoren beschrieben. Die erfindungsgemäßen Verbindungen sind in bezug auf letztgenanntes Dokument als Auswahlerfindung zu betrachten.
In der WO 99/11641 sind Phenylindolderivate mit 5-HT₂-antagonistischen Eigenschaften beschrieben.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD; z.B. WO 9524194), Angstzuständen sowie physiologischen Veränderungen, die mit Angstzuständen einhergehen wie z.B. Tachycardie, Tremor oder Schwitzen (z.B. EP 319962), Panikattacken, Psychosen, Schizophrenie, Anorexie, wahnhaften Zwangsvorstellungen, Agoraphobie, Migräne, der Alzheimer Krankheit, Schlafstörungen wie auch Schlafapnoe, tardiver Dyskinesien, Lernstörungen, altersabhängiger Erinnerungsstörungen, Essstörungen wie Bulimie, Drogenmissbrauch wie z.B. von Alkohol, Opiaten, Nikotin, Psychostimulantien wie z.B. Kokain oderAmphetaminen (z.B. US 6004980), Sexualfunktionsstörungen, Schmerzuzuständen aller Art und Fibromyalgie (z.B. WO 9946245).
Die Verbindungen der Formel I eignen sich zur Behandlung extrapyramidaler Nebenwirkungen (extrapyramidal side effects EPS) bei der neuroleptischen Drogentherapie. EPS ist gekennzeichnet durch Parkinson-ähnliche Syndrome, Akathisie und dystonische Reaktionen (z.B. EP 337136). Weiter sind sie geeignet zur Behandlung der nervösen Anorexie, Angina, Reynaud's Phänomen, koronaren Vasospasmen, bei der Prophylaxe von Migräne (z.B. EP 208235), Schmerz und Neuralgien (z.B. EP 320983), zur Behandlung des Rett-Syndroms mit autistischen Charakterzügen, des Asperger-Syndroms, des Autismus und autistischen Störungen, bei Konzentrationsmangelzuständen, Entwicklungsstörungen, Hyperaktivitätszuständen mit mentaler Unterentwicklung und stereotypen Verhaltenszuständen (z.B. WO 9524194).

Desweiteren sind sie geeignet zur Behandlung von endokrinen Erkrankungen wie Hyperprolactinaemie, ferner bei Vasospasmen, thrombotischen Erkrankungen (z.B. WO 9946245), Hypertension und gastrointestinalen Erkrankungen.
Ferner sind sie geeignet zur Behandlung cardiovaskulärer Erkrankungen sowie extrapyramidaler Symptome wie in der WO 99/11641 auf Seite 2, Zeile 24-30 beschrieben.
Die erfindungsgemäßen Verbindungen eignen sich weiter zur Verminderung des Augeninnendruckes und zur Glaucombehandlung.
Sie sind auch zur Prophylaxe und Behandlung von Vergiftungserscheinungen bei der Gabe von Ergovalin bei Tieren geeignet.
Die Verbindungen eignen sich weiterhin zur Behandlung von Erkrankungen des Herz-Kreislaufsystems (WO 99/11641, Seite 3, Zeile 14-15). Die erfindungsgemäßen Verbindungen können auch zusammen mit anderen Wirkstoffen in der Behandlung der Schizophrenie eingesetzt werden. Als andere Wirkstoffe kommen die in der WO 99/11641 auf Seite 13, Zeile 20-26 genannten Verbindungen in Frage.

Ferner können sie als Zwischenprodukte zur Herstellung weiterer Arzneimittelwirkstoffe eingesetzt werden.

Gegenstand der Erfindung sind die N-(lndolcarbonyl-)piperazinderivate der Formel I sowie ihre physiologisch unbedenklichen Säureadditionssalze. Gegenstand der Erfindung sind auch die Solvate, z.B. Hydrate oder Alkoholate, dieser Verbindungen.

Gegenstand der Erfindung sind dementsprechend die Verbindungen der Formel I sowie ein Verfahren zur Herstellung von Verbindungen der Formel I gemäß Anspruch 1.

Das Verfahren zur Herstellung von Verbindungen der Formel I gemäß Anspruch 1, ist dadurch gekennzeichnet, daß man
a) eine Verbindung der Formel II 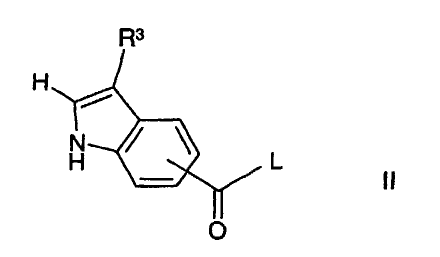 worin L Cl, Br, I, Alkylsulfonyloxy mit 1-6 C-Atomen, Arylsulfonyloxy mit 6-10 C-Atomen, Trichlormethoxy, Alkoxy oder Phenoxy bedeutet,
   und R³ die in Anspruch 1 angegebene Bedeutung hat,
   mit einer Verbindung der Formel III 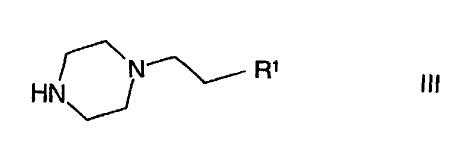 worin R¹ die in Anspruch 1 angegebene Bedeutung hat,
   umsetzt,
   oder
b) eine Verbindung der Formel IV 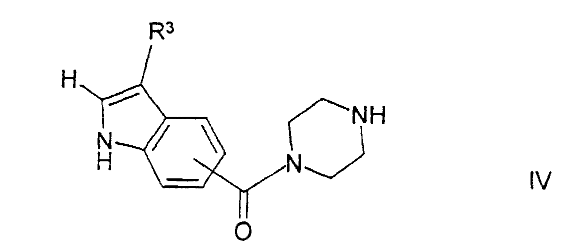 worin R³ die in Anspruch 1 angegebene Bedeutung hat,
   mit einer Verbindung der Formel V

   L-CH₂-CH₂-R¹ V

   worin L Cl, Br, I oder eine freie oder reaktionsfähig funktionell abgewandelte OH-Gruppe bedeutet, und R¹ die in Anspruch 1 angegebene Bedeutung hat,
   umsetzt,
   oder
c) gegebenenfalls einen der Reste R¹ und/oder R³ in einen anderen Rest R¹ und/oder R³ umwandelt, indem man beispielsweise eine OA-Gruppe unter Bildung einer OH-Gruppe spaltet und/oder eine CHO-Gruppe in eine CN-Gruppe umwandelt,
und/oder eine erhaltene Base der Formel I durch Behandeln mit einer Säure in eines ihrer Salze umwandelt.

Gegenstand der Erfindung sind auch die Verbindungen der Formel I gemäß Anspruch 1, sowie ihrer physiologisch unbedenklichen Salze und Solvate als Arzneimittel.

Gegenstand der Erfindung sind insbesondere die Verbindungen der Formel I gemäß Anspruch 1, sowie ihrer physiologisch unbedenklichen Salze und Solvate als Arzneimittel mit 5-HT_{2A}-Rezeptor-antagonistischer Wirkung.

Gegenstand der Erfindung sind auch die Verbindungen der Formel I sowie deren Enantiomere sowie Diastereomere und deren Salze.

Für alle Reste, die mehrfach auftreten, wie z.B. A oder Hal, gilt, daß deren Bedeutungen unabhängig voneinander sind.

Der Rest A bedeutet Alkyl und hat 1 bis 6, vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Acyl hat vorzugsweise 1-6 C-Atome und bedeutet z.B. Formyl, Acetyl, Propionyl, Butyryl, ferner Trifluoracetyl.

Alkylen ist Propylen, Butylen oder Pentylen.
OA ist vorzugsweise Methoxy, ferner auch Ethoxy, n-Propoxy, Isopropoxy, n-Butoxy, Isobutoxy, sek.-Butoxy oder tert.-Butoxy.

Hal bedeutet Fluor, Chlor, Brom oder lod, insbesondere Fluor oder Chlor.

R¹ ist unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Hydroxyphenyl, o-, m- oder p-Nitrophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-2-Chlor-4-methyl-, 2-Chlor-5-methyl-, 2-Chlor-6-methyl-, 2-Methyl-3-chlor-2-Methyl-4-Chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2,3,4-, 2,3,5-, 2,3,6-, 2,4,6- oder 3,4,5-Trichlorphenyl, 2,46-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-lodphenyl, 2-Nitro-4-methoxyphenyl, 2,5-Dimethoxy-4-nitrophenyl, 2-Methyl-5-nitrophenyl, 2,4-Dimethyl-3-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl oder 2,4,6-Triisopropylphenyl.

R¹ ist auch Het¹.
Het¹ ist vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder - 5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazoiyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl.

R¹ bedeutet ganz besonders bevorzugt Phenyl, p-Chlorphenyl, p-Fluorphenyl,Thiophen-2-yl, 5-Chlor-thiophen-2-yl, 2,5-Dichlor-thiophen-3-yl und 2- oder 3-Furyl.

R³ ist vorzugsweise unsubstituiertes, vorzugsweise - wie angegeben - monosubstituiertes Phenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-. m- oder p-tert.-Butylphenyl, o-, m- oder p-Trifluormethylphenyl, o-, m- oder p-Hydroxyphenyl, o-, m- oder p-Nitrophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-lodphenyl, 2-Nitro-4-methoxyphenyl, 2,5-Dimethoxy-4-nitrophenyl, 2-Methyl-5-nitrophenyl, 2,4-Dimethyl-3-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylpnenyi, 3-Brom-6-methoxyphenyl, 3-Chlor-6-methoxyphenyl, 2-Methoxy-5-methylphenyl oder 2,4,6-Triisopropylphenyl.

R³ ist auch Het².
Het² ist vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-lsoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazolyl,
R³ bedeutet ganz besonders bevorzugt Phenyl, p-Chlorphenyl, p-Fluorphenyl,Thiophen-2-yl, 5-Chlor-thiophen-2-yl, 2,5-Dichlor-thiophen-3-yl und 2- oder 3-Furyl.

R², R⁴, R⁵, R⁶ bedeuten jeweils unabhängig voneinander vorzugsweise H, Hal, Alkyl mit 1-6 C-Atomen, Alkoxy mit 1-6 C-Atomen oder Hydroxy, ganz besonders bevorzugt H, Hal oder Alkyl mit 1-4 C-Atomen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ig ausgedrückt werden, die der Formel I entsprechen und worin die nicht näher bezeichneten Reste die bei der Formel I angegebene Bedeutung haben, worin jedoch
in la
   - R¹: Phenyl bedeutet;
in Ib
   - R¹: unsubstituiertes oder einfach durch Hal substituiertes Phenyl bedeutet;
in Ic
   - R¹: einfach durch Hal substituiertes Phenyl oder Het¹ bedeutet;
in Id
   - R¹: unsubstituiertes oder einfach durch Hal substituiertes Phenyl oder Het¹
   bedeutet;
in le
   - R¹: unsubstituiertes oder einfach durch Hal substituiertes Phenyl oder Het¹,
   - Het¹: unsubstituiertes oder ein- oder zweifach durch Hal oder A substituiertes ungesättigtes heterocyclisches Ringsystem, welches eines oder zwei gleiche oder verschiedene Heteroatome wie Stickstoff, Sauerstoff und Schwefel enthält,
   bedeutet;
in If
   - R¹: unsubstituiertes oder einfach durch Hal substituiertes Phenyl oder Het¹,
   - Het¹: unsubstituiertes oder ein- oder zweifach durch Hal oder A substituiertes ungesättigtes heterocyclisches Ringsystem, welches eines oder zwei gleiche oder verschiedene Heteroatome wie Stickstoff, Sauerstoff und Schwefel enthält,
   - R³: unsubstituiertes oder einfach durch Hal substituiertes Phenyl oder Het²,
   bedeutet;
in Ig
   - R¹: unsubstituiertes oder einfach durch Hal substituiertes Phenyl oder Het¹,
   - Het¹: unsubstituiertes oder ein- oder zweifach durch Hal oder A substituiertes ungesättigtes heterocyclisches Ringsystem, welches eines oder zwei gleiche oder verschiedene Heteroatome wie Stickstoff, Sauerstoff und Schwefel enthält,
   - R³: unsubstituiertes oder einfach durch Hal substituiertes Phenyl oder Het²,
   - Het²: unsubstituiertes oder ein- oder zweifach durch Hal oder A substituiertes ungesättigtes heterocyclisches Ringsystem, welches ein Heteroatom wie Stickstoff, Sauerstoff und Schwefel enthält
   bedeutet.

Die Verbindungen der Formel I und auch die Ausgangsstoffe zu ihrer Herstellung werden im übrigen nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York;) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen.

In den Verbindungen der Formel II und V ist der Rest L vorzugsweise Cl oder Br; er kann jedoch auch I, OH oder auch bevorzugt eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalinsulfonyloxy) oder auch Trichlomethoxy, Alkoxy, wie z.B. Methoxy, Ethoxy, Propoxy oder Butoxy, ferner auch Phenoxy.

Die Verbindungen der Formel I können vorzugsweise erhalten werden, indem man Verbindungen der Formel II mit Verbindungen der Formel III umsetzt.
Die Ausgangsstoffe der Formeln II und III sind in der Regel bekannt; die nicht bekannten Verbindungen der Formeln II und III können leicht analog zu den bekannten Verbindungen hergestellt werden.

Die Umsetzung der Verbindungen II und III verläuft nach Methoden, wie sie für die Alkylierung bzw. Acylierung von Aminen aus der Literatur bekannt sind. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xyloi; Ketone wie Aceton, Butanon; Alkohole wie Methanol, Ethanol, Isopropanol, N-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Desweiteren kann man Verbindungen der Formel I herstellen, indem man Amine der Formel IV mit einer Komponte der Formel V enthaltend den Rest R¹ umsetzt.
Die jeweiligen Komponenten sind in der Regel bekannt oder können wie schon beschrieben nach bekannten Verfahren hergestellt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstorfsäuren wie Chlorwasserstorfsäure oder Bromwasserstorfsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung sind weiterhin die erfindungsgemäßen Arzneimittel mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Gegenstand der Erfindung ist auch eine pharmazeutische Zubereitung, enthaltend mindestens ein erfindungsgemäßes Arzneimittel sowie gegebenenfalls Träger- und/oder Hilfsstoffe und gegebenenfalls andere Wirkstoffe.
Hierbei können die Arzneimittel zusammen mit mindestens einem festen. flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung ist weiterhin die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung.

Gegenstand der Erfindung ist auch die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Die pharmazeutischen Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Dabei werden die erfindungsgemäßen Substanzen in der Regel in Analogie zu bekannten Präparaten verabreicht, vorzugsweise in Dosierungen zwischen etwa 0,1 und 500 mg, insbesondere zwischen 5 und 300 mg pro Dosierungseinheit. Die tägliche Dosierung liegt vorzugsweise zwischen etwa 0.01 und 250 mg/kg, insbesondere zwischen 0,02 und 100 mg/kg Körpergewicht.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, engt ein und reinigt durch Chromatographie an Kieselgel und/oder durch Kristallisation.

### Beispiel A1

Herstellung einer Suspension von 5-HT_{2A} Rezeptoren:
Frontaler Rattencortex wird in eiskaltem Puffer homogenisiert. Das Homogenat wird 10 Minuten bei 4°C und 50000 X zentrifugiert. Das Pellet wird in 2,5 ml eiskaltem Trispuffer resuspendiert, mit 10 ml zusätzlichem Puffer aufgefüllt und wie beschrieben zentrifugiert. Danach wird das Pellet in Puffer resuspendiert und zu einem Homogenat verdünnt, das 60 mg Material/ml enthält.
In die Inkubationsröhrchen werden 0,1 ml der Suspension, 100 µl einer 5 nM Lösung von [³H]Ketanserin, 100 µl einer Lösung der Testverbindung (Konzentration im Bereich von 10⁻⁵ bis 10⁻¹⁰ Mol pro Liter) gegeben und mit Puffer auf 1 ml aufgefüllt. Die Röhrchen werden 15 Minuten bei 37 °C inkubiert. Nach Abbrechen der Inkubation durch Eintauchen der Röhrchen in ein Eisbad wird die gekühlte Suspension durch ein Glasfilter unter Vakuum filtriert. Die Filter werden 3x mit 5 ml kaltem Puffer gewaschen und dann in Szintillationsröhrchen überführt. Die Filter werden mittels Flüssigszintillations-Spektrometrie in 8 ml Triton-X-Szintillatorflüssigkeit analysiert.

### Beispiel 1

Eine Lösung von 4-Carboxy-3-(4-chlorbenzoyl)-indol und 2-Chlor-1-methylpyridiniumjodid in N-Methylpyrrolidon (NMP) wird mit einer Lösung von 4-Phenethyl-piperazin und Ethyl-diisopropylamin (EDIPA) in NMP versetzt und 3 Stunden bei Raumtemperatur nachgerührt. Man arbeitet wie üblich auf und erhält das Rohprodukt. Dieses wird in Aceton gelöst und mit wässeriger Salzsäure wird das Hydrochlorid ausgefällt. Nach Trocknung erhält man [3-(4-Chlorbenzoyl)-1*H*-indol-4-yl]-(4-phenethyl-piperazin-1-yl)-methanon, Hydrochlorid, Hydrat F. 187,4°

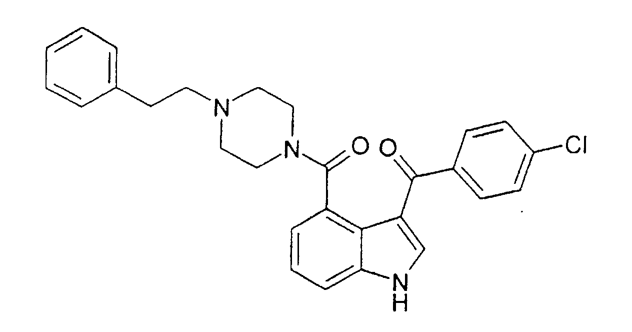

Analog erhält man die nachstehenden Verbindungen
[3-(4-Chlorbenzoyl)-1*H*-indol-4-yl]-[4-(4-fluorphenethyl)-piperazin-1-yl]-methanon,
[3-(4-Chlorbenzoyl)-1*H*-indol-4-yl]-[4-(thiophen-2-yl)-piperazin-1-yl]-methanon,
[3-(4-Chlorbenzoyl)-1*H*-indol-4-yl]-[4-(5-chlor-thiophen-2-yl)-piperazin-1-yl]-methanon,
[3-(4-Chlorbenzoyl)-1*H*-indol-4-yl]-[4-(thiophen-3-yl)-piperazin-1-yl]-methanon,
[3-(4-Chlorbenzoyl)-1*H*-indol-4-yl]-[4-(2,5-dichlor-thiophen-3-yl)-piperazin-1-yl]-methanon,
[3-(4-Chlorbenzoyl)-1*H*-indol-4-yl]-[4-(furan-2-yl)-piperazin-1-yl]-methanon.

[3-(4-Fluorbenzoyl)-1*H*-indol-4-yl]-(4-phenethyl-piperazin-1-yl)-methanon, Hydrochlorid, F. 139-141°;
[3-(4-Fluorbenzoyl)-1*H*-indol-4-yl]-[4-(4-fluorphenethyl)-piperazin-1-yl]-methanon,
[3-(4-Chlorbenzoyl)-1*H*-indol-4-yl]-[4-(4-fluorphenethyl)-piperazin-1-yl]-methanon, Hydrochlorid, F. 232°;
[3-(4-Fluorbenzoyl)-1*H*-indol-4-yl]-[4-(thiophen-2-yl)-piperazin-1-yl]-methanon,
[3-(4-Fluorbenzoyl)-1*H*-indol-4-yl]-[4-(5-chlor-thiophen-2-yl)-piperazin-1-yl]-methanon,
[3-(4-Fluorbenzoyl)-1*H*-indol-4-yl]-[4-(thiophen-3-yl)-piperazin-1-yl]-methanon,
[3-(4-Fluorbenzoyl)-1*H*-indol-4-yl]-[4-(2,5-dichlor-thiophen-3-yl)-piperazin-1-yl]-methanon,
[3-(4-Fluorbenzoyl)-1*H*-indol-4-yl]-[4-(furan-2-yl)-piperazin-1-yl]-methanon,

[3-(4-Fluorbenzoyl)-1*H*-indol-5-yl]-(4-phenethyl-piperazin-1-yl)-methanon, Hydrochlorid, F. 139-141°;
[3-(4-Chlorbenzoyl)-1*H*-indol-5-yl]-(4-phenethyl-piperazin-1-yl)-methanon, F. 168,5°;
[3-(4-Fluorbenzoyl)-1*H*-indol-5-yl]-[4-(4-fluorphenethyl)-piperazin-1-yl]-methanon, Hydrochlorid, F. 198-200°;
[3-(4-Fluorbenzoyl)-1*H*-indol-5-yl]-[4-(thiophen-2-yl)-piperazin-1-yl]-methanon,
[3-(4-Fluorbenzoyl)-1*H*-indol-5-yl]-[4-(5-chlor-thiophen-2-yl)-piperazin-1-yl]-methanon,
[3-(4-Fluorbenzoyl)-1*H*-indol-5-yl]-[4-(thiophen-3-yl)-piperazin-1-yl]-methanon,
[3-(4-Fluorbenzoyl)-1*H*-indol-5-yl]-[4-(2,5-dichlor-thiophen-3-yl)-piperazin-1-yl]-methanon,
[3-(4-Fluorbenzoyl-2-yl)-1*H*-indol-5-yl]-[4-(furan-2-yl)-piperazin-1-yl]-methanon,

[3-(Furan-2-yl)-1*H*-indol-4-yl]-(4-phenethyl-piperazin-1-yl)-methanon,
[3-(Furan-2-yl)-1*H*-indol-4-yl]-[4-(4-fluorphenethyl)-piperazin-1-yl]-methanon,
[3-(Furan-2-yl)-1*H*-indol-4-yl]-[4-(thiophen-2-yl)-piperazin-1-yl]-methanon,
[3-(Furan-2-yl)-1*H*-indol-4-yl]-[4-(5-chlor-thiophen-2-yl)-piperazin-1-yl]-methanon,
[3-(Furan-2-yl)-1*H*-indol-4-yl]-[4-(thiophen-3-yl)-piperazin-1-yl]-methanon,
[3-(Furan-2-yl)-1*H*-indol-4-yl]-[4-(2,5-dichlor-thiophen-3-yl)-piperazin-1-yl]-methanon,
[3-(Furan-2-yl)-1*H*-indol-4-yl]-[4-(furan-2-yl)-piperazin-1-yl]-methanon,

[3-(Furan-2-yl)-1*H*-indol-5-yl]-(4-phenethyl-piperazin-1-yl)-methanon, Hydrochlorid, F. 268-269°;
[3-(Furan-2-yl)-1*H*-indol-5-yl]-[4-(4-fluorphenethyl)-piperazin-1-yl]-methanon,
[3-(Furan-2-yl)-1*H*-indol-5-yl]-[4-(thiophen-2-yl)-piperazin-1-yl]-methanon,
[3-(Furan-2-yl)-1*H*-indol-5-yl]-[4-(5-chlor-thiophen-2-yl)-piperazin-1-yl]-methanon,
[3-(Furan-2-yl)-1*H*-indol-5-yl]-[4-(thiophen-3-yl)-piperazin-1-yl]-methanon,
[3-(Furan-2-yl)-1*H*-indol-5-yl]-[4-(2,5-dichlor-thiophen-3-yl)-piperazin-1-yl]-methanon,
[3-(Furan-2-yl)-1*H*-indol-5-yl]-[4-(furan-2-yl)-piperazin-1-yl]-methanon,

[3-(Thiophen-2-yl)-1*H*-indol-4-yl]-(4-phenethyl-piperazin-1-yl)-methanon,
[3-(Thiophen-2-yl)-1*H*-indol-4-yl]-[4-(4-fluorphenethyl)-piperazin-1-yl]-methanon.
[3-(Thiophen-2-yl)-1*H*-indol-4-yl]-[4-(thiophen-2-yl)-piperazin-1-yl]-methanon,
[3-(Thiophen-2-yl)-1*H*-indol-4-yl]-[4-(5-chlor-thiophen-2-yl)-piperazin-1-yl]-methanon,
[3-(Thiophen-2-yl)-1*H*-indol-4-yl]-[4-(thiophen-3-yl)-piperazin-1-yl]-methanon,
[3-(Thiophen-2-yl)-1*H*-indol-4-yl]-[4-(2,5-dichlor-thiophen-3-yl)-piperazin-1-yl]-methanon,
(3-(Thiophen-2-yl)-1*H-*indol-4-yl]-[4-(furan-2-yl)-piperazin-1-yl]-methanon,

[3-(Thiophen-2-yl)-1*H*-indol-5-yl]-(4-phenethyl-piperazin-1-yl)-methanon, Hydrochlorid, F. 191-192°;
[3-(Thiophen-2-yl)-1*H*-indol-5-yl]-[4-(4-fluorphenethyl)-piperazin-1-yl]-methanon,
[3-(Thiophen-2-yl)-1*H*-indol-5-yl]-[4-(thiophen-2-yl)-piperazin-1-yl]-methanon,
[3-(Thiophen-2-yl)-1*H*-indol-5-yl]-[4-(5-chlor-thiophen-2-yl)-piperazin-1-yl]-methanon,
[3-(Thiophen-2-yl)-1*H*-indol-5-yl]-[4-(thiophen-3-yl)-piperazin-1-yl]-methanon,
[3-(Thiophen-2-yl)-1*H*-indol-5-yl]-[4-(2,5-dichlor-thiophen-3-yl)-piperazin-1-yl]-methanon,
[3-(Thiophen-2-yl)-1*H*-indol-5-yl]-[4-(furan-2-yl)-piperazin-1-yl]-methanon.

### Beispiel 2

Durch katalytische Hydrierung mit Wasserstoff und Pd/C in Methanol erhält man aus [3-(4-Chlorbenzoyl)-1*H*-indol-4-yl]-[4-(4-fluorphenethyl)-piperazin-1-yl]-methanon die Verbindung
[3-Benzoyl-1*H*-indol-4-yl]-[4-(4-fluorphenethyl)-piperazin-1-yl]-methanon, Hydrochlorid, F. 257°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6.8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I 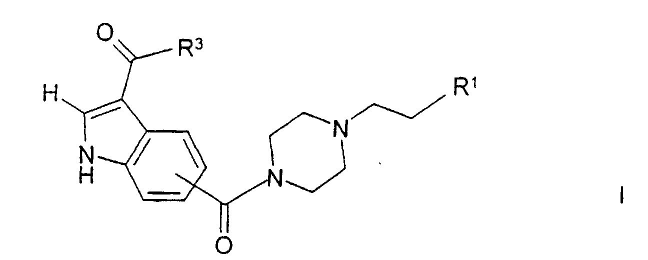 worin
R¹ einen unsubstituierten oder durch R² und/oder R⁴ substituierten Phenylrest
oder Het¹,
R², R⁴, R⁵, R⁶ jeweils unabhängig voneinander Hal, A, OA, OH, CN oder Acyl,
R³ einen unsubstituierten oder durch R⁵ und/oder R⁶ substituierten Phenylrest
oder Het²,
Het¹ ein- oder zweikerniges unsubstituiertes oder ein- oder zweifach durch Hal, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
Het² unsubstituiertes oder ein- oder zweifach durch Hal, A, OA, ungesättigtes heterocyclisches Ringsystem, welches eines oder zwei gleiche oder verschiedene Heteroatome wie Stickstoff, Sauerstoff und Schwefel enthält,
A Alkyl mit 1-6 C-Atomen,
Hal F, Cl, Br oder I,
bedeuten,
sowie deren physiologisch unbedenklichen Salze und Solvate.

2. Verfahren zur Herstellung von Verbindungen der Formel I gemäß Anspruch 1, **dadurch gekennzeichnet, daß** man
a) eine Verbindung der Formel II 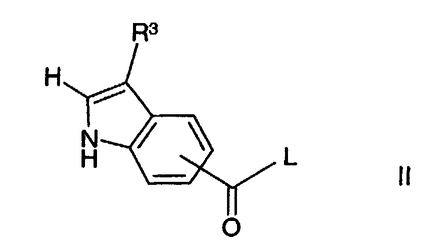 worin L Cl, Br, I, Alkylsulfonyloxy mit 1-6 C-Atomen, Arylsulfonyloxy mit 6-10 C-Atomen, Trichlormethoxy, Alkoxy oder Phenoxy bedeutet, und R³ die in Anspruch 1 angegebene Bedeutung hat,
mit einer Verbindung der Formel III 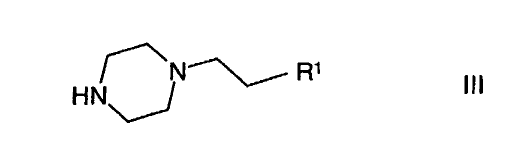 worin R¹ die in Anspruch 1 angegebene Bedeutung hat,
umsetzt,
oder
b) eine Verbindung der Formel IV 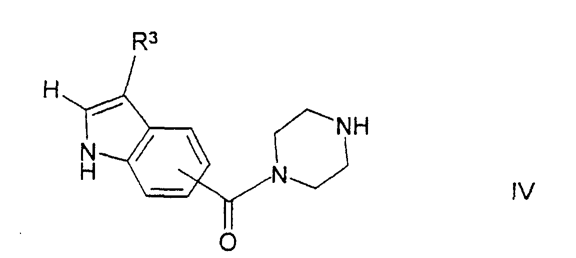 worin R³ die in Anspruch 1 angegebene Bedeutung hat,
mit einer Verbindung der Formel V
L-CH₂-CH₂-R¹ V
worin L Cl, Br, I oder eine freie oder reaktionsfähig funktionell abgewandelte OH-Gruppe bedeutet, und R¹ die in Anspruch 1 angegebene Bedeutung hat,
umsetzt,
oder
c) gegebenenfalls einen der Reste R¹ und/oder R³ in einen anderen Rest R¹ und/oder R³ umwandelt, indem man beispielsweise eine OA-Gruppe unter Bildung einer OH-Gruppe spaltet und/oder eine CHO-Gruppe in eine CN-Gruppe umwandelt
und/oder
eine erhaltene Base der Formel I durch Behandeln mit einer Säure in eines ihrer Salze umwandelt.

3. Verbindungen der Formel I gemäß Anspruch 1, sowie ihrer physiologisch unbedenklichen Salze und Solvate als Arzneimittel.

4. Verbindungen der Formel I gemäß Anspruch 1, sowie ihrer physiologisch unbedenklichen Salze und Solvate als Arzneimittel mit 5-HT_{2A}-Rezeptor-antagonistischer Wirkung.

5. Arzneimittel nach Anspruch 4 zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

6. Pharmazeutische Zubereitung, enthaltend mindestens ein Arzneimittel gemäß Anspruch 5, sowie gegebenenfalls Träger- und/oder Hilfsstoffe und gegebenenfalls andere Wirkstoffe.

7. Verwendung von Verbindungen gemäß Anspruch 1 und/oder von deren physiologisch unbedenklichen Salzen und Solvaten zur Herstellung eines Arzneimittels mit 5-HT_{2A}-Rezeptor-antagonistischer Wirkung.

8. Verwendung nach Anspruch 7 zur Herstellung eines Arzneimittels zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

## Claims

1. Compounds of the formula I 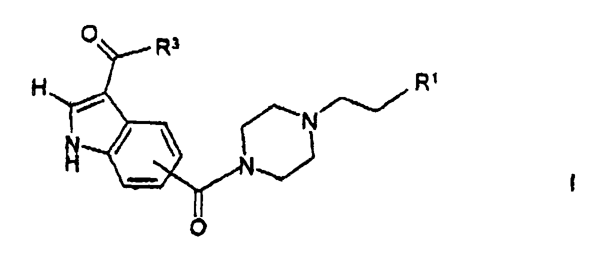 in which
R¹ is a phenyl radical which is unsubstituted or substituted by R² and/or R⁴
or Het¹,
R², R⁴, R⁵ and R⁶ are each, independently of one another, Hal, A, OA, OH, CN or acyl,
R³ is a phenyl radical which is unsubstituted or substituted by R⁵ and/or R⁶
or Het²,
Het¹ is a mono- or bicyclic unsaturated heterocyclic ring system which is unsubstituted or mono- or disubstituted by Hal, A, OA or OH and which contains one, two or three identical or different heteroatoms, such as nitrogen, oxygen and sulfur,
Het² is an unsaturated heterocyclic ring system which is unsubstituted or mono- or disubstituted by Hal, A, OA and which contains one or two identical or different heteroatoms, such as nitrogen, oxygen and sulfur,
A is alkyl having 1-6 Carbon atoms,
Hal is F, Cl, Br or I,
and physiologically acceptable salts and solvates thereof.

2. Process for the preparation of compounds of the formula I according to Claim 1, **characterised in that**
a) a compound of the formula II 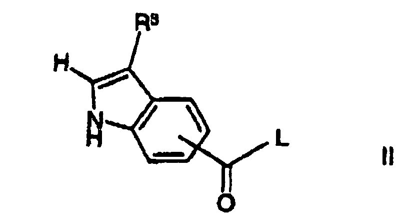 in which L is Cl, Br, I, alkylsulfonyloxy having 1-6 carbon atoms, arylsulfonyloxy having 6-10 carbon atoms, trichloromethoxy, alkoxy or phenoxy,
and R³ is defined in Claim 1,
is reacted with a compound of the formula III 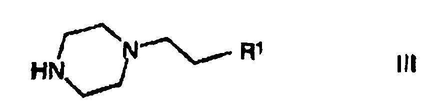 in which R¹ is as defined in Claim 1,
or
b) a compound of the formula IV 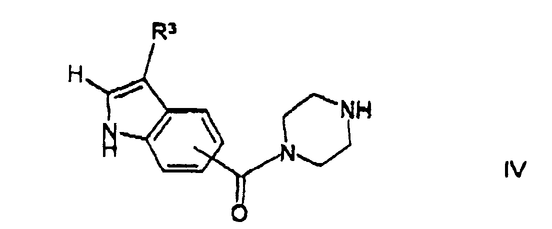 in which R³ is as defined in Claim 1,
is reacted with a compound of the formula V
L-CH₂-CH₂-R¹ V
in which L is Cl, Br, I or a free or reactively functionally modified OH group, and R¹ is as defined in Claim 1,
or
c) one of the radicals R¹ and/or R³ is, if desired, converted into another radical R¹ and/or R³ by, for example, cleaving an OA group with formation of an OH group and/or converting a CHO group into a CN group
and/or
a resultant base of the formula I is converted into one of its salts by treatment with an acid.

3. Compounds of the formula I according to Claim 1, and physiologically acceptable salts and solvates thereof as medicaments.

4. Compounds of the formula I according to Claim 1, and physiologically acceptable salts and solvates thereof as medicaments having a 5-HT_{2A} receptor-antagonistic action.

5. Medicament according to Claim 4 for the treatment of psychoses, schizophrenia, depression, neurological disorders, memory disorders, Parkinson's disease, amyotrophic lateral sclerosis, Alzheimer's disease, Huntington's disease, eating disorders, such as bulimia and nervous anorexia, premenstrual syndrome and/or for positively influencing obsessive-compulsive disorder (OCD).

6. Pharmaceutical preparation comprising at least one medicament according to Claim 5, and optionally excipients and/or adjuvants and optionally other active ingredients.

7. Use of compounds according to Claim 1 and/or of physiologically acceptable salts and solvates thereof for the preparation of a medicament having a 5-HT_{2A} receptor-antagonistic action.

8. Use according to Claim 7 for the preparation of a medicament for the treatment of psychoses, schizophrenia, depression, neurological disorders, memory disorders, Parkinson's disease, amyotrophic lateral sclerosis, Alzheimer's disease, Huntington's disease, eating disorders, such as bulimia and nervous anorexia, premenstrual syndrome and/or for positively influencing obsessive-compulsive disorder (OCD).

## Revendications

1. Composés de formule I 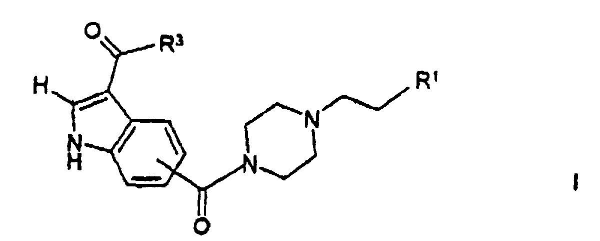 dans laquelle
R¹ désigne un radical phényle qui est non substitué ou substitué par R² et/ou R⁴
ou Hét¹,
R², R⁴, R⁵, R⁶ chacun, indépendamment l'un de l'autre, désignent Hal, A, OA, OH, CN ou acyle,
R³ désigne un radical phényle qui est non substitué ou substitué par R⁵ et/ou R⁶
ou Hét²,
Hét¹ désigne un cycle hétérocyclique mono- ou bicyclique insaturé qui est non substitué ou mono- ou disubstitué par Hal, A, OA ou OH et qui contient un, deux ou trois hétéroatomes identiques ou différents, tels que l'azote, l'oxygène et le soufre,
Hét² désigne un cycle hétérocyclique insaturé qui est non substitué ou mono- ou disubstitué par Hal, A, OA et qui contient un ou deux hétéroatomes identiques ou différents, tels que l'azote, l'oxygène et le soufre,
A désigne alkyle ayant de 1 à 6 atomes de C,
Hal désigne F, Cl, Br ou I,
et les sels et les solvats physiologiquement acceptables de ceux-ci.

2. Procédé de préparation de composés de formule I selon la revendication 1, **caractérisé en ce que**
a) un composé de formule II 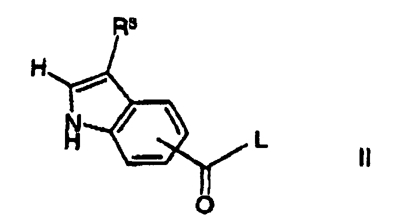 dans laquelle L désigne Cl, Br, I, alkylsulfonyloxy ayant de 1 à 6 atomes de C, arylsulfonyloxy ayant de 6 à 10 atomes de C, trichlorométhoxy, alkoxy ou phénoxy,
et R³ a la signification donnée à la revendication 1,
est réagi avec un composé de formule III 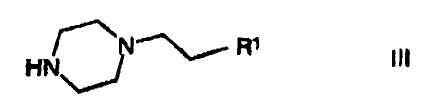 dans laquelle R¹ a la signification donnée à la revendication 1,
ou
b) un composé de formule IV 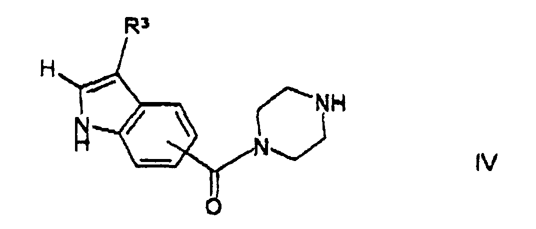 dans laquelle R³ a la signification donnée à la revendication 1,
est réagi avec un composé de formule V
L-CH₂-CH₂-R¹ V
dans laquelle L désigne Cl, Br, I ou un groupement OH libre ou modifié de façon réactive et fonctionnelle, et R¹ a la signification donnée à la revendication 1,
ou
c) l'un des radicaux R¹ et/ou R³ est éventuellement converti en un autre radical R¹ et/ou R³ au moyen, par exemple, du clivage d'un groupement OA avec la formation d'un groupement OH et/ou de la conversion d'un groupement CHO en un groupement CN
et/ou
une base résultante de formule I est convertie en l'un de ses sels par un traitement par un acide.

3. Composés de formule I selon la revendication 1, et les sels et les solvats physiologiquement acceptables de ceux-ci comme médicaments.

4. Composés de formule I selon la revendication 1, et les sels et les solvats physiologiquement acceptables de ceux-ci comme médicaments ayant une action antagoniste du récepteur de 5-HT_{2A}.

5. Médicament selon la revendication 4 pour le traitement des psychoses, de la schizophrénie, de la dépression, des troubles neurologiques, des troubles de la mémoire, de la maladie de Parkinson, de la sclérose latérale amyotrophique, de la maladie d'Alzheimer, de la maladie d'Huntington, des troubles de l'alimentation, tels que la boulimie ou l'anorexie mentale, et du syndrome prémenstruel et/ou pour influencer de façon positive le trouble obsessionnel-compulsif (TOC).

6. Préparation pharmaceutique comprenant au moins un médicament selon la revendication 5, et éventuellement des excipients et/ou des adjuvants et éventuellement d'autres ingrédients actifs.

7. Utilisation de composés selon la revendication 1 et/ou de sels et de solvats physiologiquement acceptables de ceux-ci pour la préparation d'un médicament ayant une action antagoniste du récepteur de 5-HT_{2A}.

8. Utilisation selon la revendication 7 pour la préparation d'un médicament pour le traitement des psychoses, de la schizophrénie, de la dépression, des troubles neurologiques, des troubles de la mémoire, de la maladie de Parkinson, de la sclérose latérale amyotrophique, de la maladie d'Alzheimer, de la maladie d'Huntington, des troubles de l'alimentation, tels que la boulimie ou l'anorexie mentale, et du syndrome prémenstruel et/ou pour influencer de façon positive le trouble obsessionnel-compulsif (TOC).
